# EUROPÄISCHE PATENTSCHRIFT

(11) **EP 1 173 407 B1**
(45) Veröffentlichungstag und Bekanntmachung des Hinweises auf die Patenterteilung: **15.06.2005**
(21) Anmeldenummer: 00917076.2
(22) Anmeldetag: 12.04.2000
(51) Int. Cl.: C07C 227/16, C07C 229/60, C07D 235/06

(54) **VERFAHREN ZUR NITRIERUNG VON ANILINDERIVATEN**
METHOD FOR NITRATING ANILINE DERIVATIVES
PROCEDE DE NITRATION DE DERIVES D'ANILINE

(30) Priorität: 17.04.1999 DE 19917524
(43) Veröffentlichungstag der Anmeldung: 23.01.2002
(73) Patentinhaber: Boehringer Ingelheim Pharma GmbH & Co.KG, 55218 Ingelheim am Rhein (DE)
(72) Erfinder: SCHNEIDER, Heinrich, Deceased (DE)
(74) Vertreter: Kläs, Heinz-Gerd, Dr.
(86) Internationale Anmeldenummer: PCT/EP2000/003247
(87) Internationale Veröffentlichungsnummer: WO 2000/063158

(56) Entgegenhaltungen:
- US-A- 4 882 342
- RIES, UWE J ET AL: "6-Substituted benzimidazoles as new nonpeptide angiotensin II receptor antagonists: Synthesis, biological activity, and structure-activity relationships" JOURNAL OF MEDICINAL CHEMISTRY., Bd. 36, Nr. 25, 1993, Seiten 4040-4051, XP002142236 AMERICAN CHEMICAL SOCIETY. WASHINGTON., US ISSN: 0022-2623 in der Anmeldung erwähnt
- KUBO, KEIJI ET AL: "Nonpeptide angiotensin receptor antagonists. Synthesis and biological activity of benzimidazoles" JOURNAL OF MEDICINAL CHEMISTRY., Bd. 36, Nr. 12, 1993, Seiten 1772-1784, XP002142237 AMERICAN CHEMICAL SOCIETY. WASHINGTON., US ISSN: 0022-2623
- MONGE, ANTONIO ET AL: "Hypoxia-selective agents derived from quinoxaline 1,4-di-N-oxides" JOURNAL OF MEDICINAL CHEMISTRY., Bd. 38, Nr. 10, 1995, Seiten 1786-1792, XP002142238 AMERICAN CHEMICAL SOCIETY. WASHINGTON., US ISSN: 0022-2623

## Beschreibung

Die vorliegende Erfindung betrifft insbesondere ein Verfahren zur hochregioselektiven aromatischen Nitrierung von 4-Alkanoylamino-3-alkyl-benzoesäurealkylestern in 5-Position in einem Salpetersäure-haltigen Gemisch. Gegebenenfalls befindet sich an Stelle des 3-Alkylrests ein H.

### Hintergrund der Erfindung

Die Mononitrierung von Amino-substituierten Aromaten ist technisch bedeutsam, da sie einen Weg zu Herstellung von Nitroanilinen eröffnet, die u.a. wichtige Zwischenverbindungen für die Synthese von Farbstoffen, Antioxidantien und insbesondere Pharmazeutika darstellen. Besondere Bedeutung hat dabei die Mononitrierung von Anilinderivaten in ortho-Stellung, da die entstehenden 1, 2-Nitroaniline weiter zu aromatischen Heterocyclen mit zwei Hetero-Stickstoffatomen, wie beispielsweise Benzimidazolen, umgesetzt werden können.

Leider jedoch ist die großtechnische Nitrierung von Aromaten mit verschiedenen Schwierigkeiten behaftet.

So sind die gewünschten Produkte in der Regel nicht isomerenrein zugänglich, da das Isomerengleichgewicht bei der Mononitrierung verschieden substituierter Aromaten bekanntermaßen nur wenig von den Reaktionsbedingungen beeinflußbar ist (vgl. z.B. die großtechnische Monontirierung von Toluol: K. Weissermehl, H.-J. Arpe, Industrielle organische Chemie, 3. Auflage Seite 400-401). Die Abtrennung des gewünschten Regioisomeren gestaltet sich mitunter sehr schwierig. Für die großtechnische Synthese von Pharmazeutika ist es jedoch unerläßlich, in den einzelnen Reaktionsstufen äußerst reine Produkte zu erhalten.

Eine weitere Schwierigkeit bei Nitrierungen in großtechnischem Maßstab stellt das mitunter erhebliche Sicherheitsrisiko dar. So sind auf der einen Seite die für die Nitrierung von Aromaten erforderlichen Reaktionslösungen in der Regel hochexplosiv, insbesondere dann, wenn als nitrierendes Agens konzentrierte Salpetersäure eingesetzt wird. Die Reaktionsführung ist besonders dann sehr risikoreich, wenn die Reaktionsmassenanhäufung sehr groß wird. In solchen Fällen reagieren die einzelnen Reaktanden nicht unmittelbar bei ihrer Zusammenführung, sondern zeitlich verzögert. Dadurch wird die kontinuierliche und damit kontrollierte Reaktionsführung erschwert und die Gefahr einer spontanen explosionsartigen Reaktion nimmt zu. Ein weiteres schwer kalkulierbares Sicherheitsrisiko können die aus der Reaktion hervorgehenden Produkte darstellen. Besonders prekär sind diesbezüglich di- und trinitrierte Nebenprodukte. Letztere können in großtechnischen Nitrierungsverfahren mitunter spontan entstehen, so daß sie außerhalb der Kontrolle der Verfahrensverantwortlichen liegen.

### Beschreibung der Erfindung

Daher ist es Aufgabe der vorliegenden Erfindung, ein großtechnisches Verfahren zur Mononitrierung insbesondere von 4-Alkanoylamino-3-alkyl-5-nitrobenzoesäurealkylestem zu schaffen, welches die aus dem Stand der Technik bekannten Nachteile überwindet.

Eine besondere Aufgabe der Erfindung besteht dabei darin, ein großtechnisches hochregioselektives Verfahren zur aromatischen Nitrierung von insbesondere 4-Alkanoylamino-3-alkylbenzoesäurealkylestem in 5-Position zu schaffen, bei dem die Bildung von Isomeren und gegebenenfalls von di- und /oder trinitriertem 4-Alkanoylamino-3-alkylbenzoesäurealkylestem minimiert bzw. vollständig unterdrückt wird.

Ein weiteres Ziel der vorliegenden Erfindung ist es, ein großtechnisch einsetzbares Verfahren zur aromatischen Nitrierung von 4-Alkanoylamino-3-alkyl-benzoesäurealkylestern bereit zu stellen, das eine einfache Isolierung der entstehenden 4-Alkanoylamino-3-alkyl-5-nitrobenzoesäurealkylester ermöglicht.

Schließlich ist es auch eine Aufgabe der Erfindung, 4-Alkanoylamino-3-alkyl-5-nitrobenzoesäurealkylester großtechnisch herzustellen, die weiter zu pharmakologisch aktiven Benzimidazolen, umgesetzt werden können.

### Detaillierte Beschreibung der Erfindung

Überraschenderweise wurde gefunden, daß Verbindungen der allgemeinen Formel I, insbesondere 4-Alkanoylamino-3-alkyl-5-nitrobenzoesäurealkylester der allgemeinen Formel I, 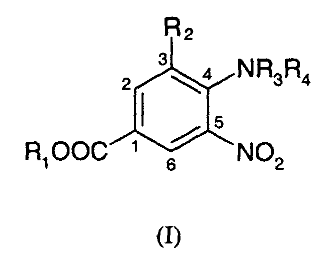 worin R₁ H oder C₁ bis C₆ alkyl;
R₂ H oder C₁ bis C₆-alkyl;
R₃ H;
R₄ H oder C₁ bis C₅-alkyl-carbonyl bedeuten
oder R₃ und R₄ zusammen Succinyl, Glutary, Adipinyl bedeuten können völlig unerwartet weitgehend isomerenrein aus Verbindungen gemäß der allgemeinen Formel II 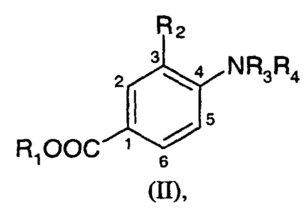 worin R₁, R₂, R₃ und R₄ die in der Formeln I genannten Bedeutung aufweisen, dadurch hergestellt werden können, daß in einem ersten Schritt Verbindungen der Formel II, insbesondere der 4-Alkanoylamino-3-alkyl-benzoesäurealkylester der Formel II in einem Gemisch aus Schwefelsäure, Salpetersäure und Wasser ― wie im Anspruch 1 angegeben - ohne dabei zu reagieren gelöst wird und in einem zweiten Schritt durch Zugabe von Salpetersäure als Nitrierungsagens zu weitgehend isomerenreinen Verbindungen der allgemeinen Formel I, insbesondere dem 4-Alkanoylamino-3-alkyl-5-nitrobenzoesäurealkylester der allgemeinen Formel I umgesetzt wird. Im ersten Schritt des Verfahrens wird das Edukt bei weniger als 10°C dem Lösungsmittel zugegeben. Während der Zugabe der Salpetersäure wird die Reaktionstemperatur bei -20°C bis + 10°C, gehalten.

Demgemäß löst das erfindungsgemäße Verfahren die gestellte Aufgabe dadurch, daß das Edukt, eine Verbindung der allgemeinen Formel II, insbesondere 4-Alkanoylamino-3-alkylbenzoesäurealkylester gemäß der allgemeinen Formel II, in einem ersten Schritt so in einem Gemisch aus Wasser, Schwefelsäure und Salpetersäure gelöst wird, daß es nicht oder weitgehend nicht reagiert. In einem zweiten Schritt wird die hochregioselektive Umsetzung zu Verbindungen der allgemeinen Formel I, insbesondere 4-Alkanoylamino-3-alkyl-5-nitrobenzoesäurealkylester der allgemeinen Formel I durch Zugabe eines Nitrierungsagens bewirkt.

Ein bevorzugtes Verfahren betrifft die Herstellung von Verbindungen der allgemeinen Formel I, insbesondere von 4-Alkanoylamino-3-alkyl-5-nitrobenzoesäurealkylester der allgemeinen Formel I, wobei die Reste wie folgt definiert sind:
R₁ H, Methyl, Ethyl, Propyl, Isopropyl, Butyl, Isobutyl, Pentyl, Hexyl;
R₂ H, Methyl, Ethyl, Propyl, Isopropyl, Butyl, Isobutyl, Pentyl, Hexyl;
R₃ H;
R₄ H, Acetyl, Propionyl, Butyryl, Valeryl, Capryl,
oder R₃ und R₄ können zusammen einen Succinyl-, Glutaryl- oder Adipinyl-Rest bilden. Besonders bevorzugt sind 4-Alkanoylamino-3-alkyl-5-nitrobenzoesäurealkylester der allgemeinen Formel I mit R₁ Methyl; R₂ Methyl; R₃ Butyryl und R₄ H.
Demzufolge werden bevorzugt 4-Alkanoylamino-3-alkyl-benzoesäurealkylester der allgemeinen Formel II eingesetzt bei dem die Reste R₁, R₂, R₃ und R₄ die obige Bedeutung haben.
Die verwendeten Säuren werden bevorzugt konzentriert eingesetzt, insbesondere Schwefelsäure als 96 Gew.% Schwefelsäure und Salpetersäure als ca. 98-100 Gew.% Salpetersäure.

Die Komponenten Salpetersäure, Schwefelsäure und Wasser für das Gemisch zum Lösen des Edukts im ersten Schritt des Verfahrens werden in einem molaren Verhältnis von 1,5-2,5 mol Salpetersäure zu 1,5-2,5 mol Schwefelsäure zu 2-6 mol Wasser pro mol umzusetzendem Edukt eingesetzt. Ein bevorzugtes molares Verhältnis der Komponenten beträgt 1,8 - 2,2 mol Salpetersäure zu 1,8 - 2,2 mol Schwefelsäure zu 2,5 - 4,0 mol Wasser pro mol umzusetzendem Edukt. Ein besonders bevorzugtes molares Verhältnis der Komponenten beträgt 2 mol Salpetersäure zu 2 mol Schwefelsäure zu 3 mol Wasser pro mol umzusetzendem Edukt.

Im ersten Schritt des Verfahrens wird das Edukt kontinuierlich oder portionsweise bei weniger als 10°C, bevorzugt bei weniger als 0°C dem Lösungsmittel zugegeben und darin gelöst. Die bevorzugte Temperatur liegt zwischen -20°C bis 10°C, ganz besonders bevorzugt zwischen -10°C und - 5°C.

Das so in dem oben beschriebenen Gemisch gelöste Edukt wird in einem zweiten Schritt des Verfahrens nitriert.

Das Nitrierungsagens ist Salpetersäure, insbesondere 98 - 100 Gew.% Salpetersäure. An dieser Stelle sei daran erinnert, daß die Menge und Konzentration der im ersten Schritt für das Gemisch zum Lösen des Edukts verwendete Salpetersäure zum Nitrieren des Edukts nicht ausreicht, so daß die zum Nitrieren notwendige Menge Salpeteräure in diesem zweiten Schritt durch Zugabe weiterer Salpetersäure langsam eingestellt werden muß.
Die Menge der für die Nitrierung notwendigen Salpetersäure variiert in einem Toleranzband von 6 bis 10 mol pro mol zu nitrierendes Edukt, bevorzugt 7 - 9 mol pro mol Edukt, ganz besonders bevorzugt ca. 8 mol pro mol Edukt. Die Zugabe erfolgt kontinuierlich oder portionsweise über einen Zeitraum von mehr als 2 Stunden. Bevorzugte Zugabezeiten sind zwei bis zu 6 Stunden, insbesondere bevorzugt 3 bis 4 Stunden.
Während der Zugabe wird die Reaktionstemperatur bei-20°C bis + 10°C, bevorzugt bei -5°C bis +5°C gehalten.

Nach beendeter Reaktion wird der Ansatz auf Wasser gegeben. Um Folgereaktionen des entstandenen Produkts mit Wasser zu minimieren, bzw. gänzlich zu vermeiden, sollte darauf geachtet werden, daß die wäßrige Mischung eine Temperatur von +50°C nicht übersteigt, die obere Temperaturbereich der Mischung liegt bevorzugt zwischen 30°C und +40°C.

Als Produkt gemäß dem erfindungsgemäßen Verfahren wird weitgehend isomerenreiner 4-Alkanoylamino-3-alkyl-5-nitrobenzoesäurealkylester der allgemeinen Formel I erhalten. Unter der Bezeichnung isomerenrein bzw. weitgehend isomerenrein werden im vorliegenden Kontext 4-Alkanoylamino-3-alkyl-5-nitrobenzoesäurealkylester der allgemeinen Formel I verstanden, die gänzlich oder weitgehend frei von 4-Alkanoylamino-3-alkyl-6-nitrobenzoesäurealkylester und / oder 4-Alkanoylamino-3-alkyl-2-nitrobenzoesäurealkylester sind, wobei die Substituenten R₁, R₂, R₃, und R₄ in den drei zuletzt genannten Verbindungen identisch sind. Der Gesamtanteil der 2- und 6-Nitroisomeren beläuft sich dabei auf weniger als 2 mol% bezogen auf das Hauptprodukt.

Ein weiterer Vorteil des beschriebenen Verfahrens liegt in dessen vergleichsweiser hoher Sicherheit. Dies spiegelt sich auch darin wider, daß bei einer Dosierzeit der Salpetersäure im zweiten Schritt von 3 h die Reaktionsmassenanhäufung bei ca. 5 -10% liegt. D.h. die Nitrierung des Edukts findet so schnell statt, daß während des Zutropfens der Salpetersäure im Mittel nur maximal 5-10 % der umzusetzenden Reaktionspartner nicht umgesetzt vorliegen. Das hat den Vorteil, daß sich bei einem Ausfall des Kühlmittels oder sonstiger Energien die Reaktionsmasse nur soweit selbst erwärmen kann, daß sie sich noch nicht im Bereich einer sich selbst beschleunigenden Zersetzung befindet.

Das hat den Vorteil, daß sich bei einem Ausfall des Kühlmittels oder sonstiger Energien die Reaktionsmasse nur soweit selbst erwärmen kann, daß sie sich noch nicht im Bereich einer sich selbst beschleunigenden Zersetzung befindet.

Bevorzugt ist ein solches Nitrierverfahren, welches dadurch gekennzeichnet ist, daß die Komponenten des Lösungsmittelgemisches des ersten Schritts in einem molaren Verhältnis von 1,8 - 2,2 mol Salpetersäure zu 1,8 - 2,2 mol Schwefelsäure zu 2,5 - 4,0 mol Wasser pro mol der Verbindung der allgemeinen Formel II, insbesondere 4-Alkanoylamino-3-alkylbenzoesäurealkylester der Formel II stehen und im zweiten Schritt 7 - 9 mol Salpetersäure pro mol der Verbindung der allgemeinen Formel II, insbesondere 4-Alkanoylamino-3-alkylbenzoesäurealkylester der Formel II hinzugegeben werden.

Insbesondere bevorzugt ist ein solches Nitrierverfahren, bei dem das Verhältnis der Komponenten des Lösungsmittelgemisches des ersten Schritts 2 mol Salpetersäure zu 2 mol Schwefelsäure zu 3 mol Wasser pro mol der Verbindungen der allgemeinen Formel II, insbesondere 4-Alkanoylamino-3-alkyl-benzoesäurealkylester der Formel II beträgt und im zweiten Schritt 8 mol Salpetersäure pro mol der Verbindung der allgemeinen Formel II, insbesondere 4-Alkanoylamino-3-alkyl-benzoesäurealkylester der Formel II hinzugegeben werden.

In einer gleichfalls bevorzugte Ausführung eines solchen Nitrierverfahrens wird der 4-Alkanoylamino-3-alkyl-benzoesäurealkylester der Formel II im ersten Schritt bevorzugt bei -20 bis + 10°C in das Lösungsmittelgemisch eingetragen.

In einer weiterhin bevorzugte Ausführung eines solchen Nitrierverfahrens erfolgt die Zugabe der Salpetersäure im zweiten Schritt bei einer Temperatur zwischen - 10 und + 10°C, bevorzugt bei -5 bis + 5°C.

In allen Ausführungsbeispielen sind insbesondere solche Nitrierverfahren bevorzugt, bei denen die Reste R₁, R₂, R₃ und R₄ die folgende Bedeutung haben:
R₁ H, Methyl, Ethyl, Propyl, Isopropyl, Butyl, Isobutyl, Pentyl, Hexyl; R₂ H, Methyl, Ethyl, Propyl, Isopropyl, Butyl, Isobutyl, Pentyl, Hexyl; R₃ H; R₄H, Acetyl, Propionyl, Butyryl, Valeryl, Capryl oder R₃ und R₄ bilden zusammen einen Succinyl-, Glutaryl- oder Adipinyl-Rest. Besonders bevorzugte sind solche Verbindungen mit R₁ Methyl; R₂ Methyl; R₃ H und R₄ Butyryl.

Die durch die obige Beschreibung herstellbaren 4-Alkanoylamino-3-alkyl-5-nitrobenzoesäurealkylester der allgemeine Formel I stellen wichtige Bausteine für die Synthese von Benzimidazolen dar, besonders diejenigen der Formel III 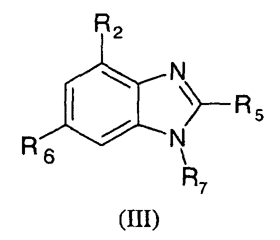 mit den Resten R₂ Methyl; R₅ Propyl; R₆ N-Methylbenzimidazol-2-yl- oder COOR₁, wobei R₁ wie in Formel 1 definiert ist und R₇ H, zu denen sie durch einfache Folgereaktionen umgesetzt werden können.

Die so zugänglichen oder weiter derivatisierten Benzimidazole und deren Salze sind wegen ihrer pharmakologischen Vielfältigkeit von hohem Interesse. Sie finden z.B. zur Behandlung der Hypertonie und Herzinsuffizienz, zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Neuropathie, des Glauder Hypertonie und Herzinsuffizienz, zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Neuropathie, des Glau koms, von gastrointestinalen und Blasenerkrankungen Verwendung und können darüberhinaus anthelmintische Wirkung haben.

Die Verbindungen der allgemeinen Formel I, insbesondere die 4-Alkanoylamino-3-alkyl-5-nitrobenzoesäurealkylester der allgemeine Formel I können z.B. gemäß den im Journal of Medicinal Chemistry 1993, Vol. 36, No. 25 Seite 4040 - 4051 beschriebenen Verfahren zu Benzimidazolen und deren Derivaten umgesetzt werden. Insbesondere die Verbindung der allgemeine Formel I mit R₁ Methyl; R₂ Methyl; R₃ H; R₄ Butyryl kann gemäß dem auf Seite 4050 beschriebenen Reaktionsschema V durchgeführt werden. Weitere Umsetzungsmöglichkeiten finden sich z.B. im europäischen Patent EP 0 502 314 B1. Als Beispiel sei besonders auf die Seite 11, Zeile 23 ff der EP 0 502 314 B1 beschriebenen Umsetzung von nitriertem 3-Methyl-4-n-butanoyl-aminobenzoesäure-methylester hingewiesen.

### Beispiele

In einem 500 1-Email-Rührwerksapparat werden 27,5 l Wasser und 122,5 kg Schwefelsäure 96 % vorgelegt und auf -10°C abgekühlt. Zunächst werden bei ca. -10 bis 0°C 76,4 kg Salpetersäure 99 % zudosiert und dann innerhalb von 1 - 2 h 141 kg 4-Butyrylamino-3-methylbenzoesäuremethylester bei -10 bis -5°C über das Mannloch eingetragen. Danach werden bei -5 bis +5°C innerhalb von 3 - 4 h weitere 305,5 kg Salpetersäure 99 % zudosiert. Man rührt noch gut 30 - 60 min nach und läßt dabei die Temperatur auf +5 bis 10°C ansteigen. Dieses Gemisch wird dann bei 35 - 40°C unter Rühren in einen mit 564 l Wasser beladenen 1200 1-Email-Rührwerksapparat gegeben. Man spült mit 141 l Wasser nach und kühlt auf 15 - 20°C ab. Das ausgefallene Produkt wird abgeschleudert, mit Wasser gewaschen und getrocknet. Ausbeute : 153 kg 4-Butyrylamino-3-methyl-5-nitrobenzoesäuremethylester, praktisch isomerenfrei (91 % d. Th.).

### Vergleichsreaktionen mit aus dem Stand der Technik bekannten Verfahren

### Vergleichsbeispiel A

In einem 500 1-Email-Rührwerksapparat werden 45 1 Wasser und 130 kg Schwefelsäure 96 % vorgelegt. Bei max. 20°C werden 209,5 kg Salpetersäure 99 % zudosiert, auf ca. -10°C abgekühlt und dann bei -10 bis -5°C 35,3 kg 4-Butyrylamino-3-methylbenzoesäuremethylester über das Mannloch eingetragen. Man rührt noch gut 15 min nach, läßt die Temperatur auf 0°C ansteigen und rührt nochmals gut 1 h. Das Gemisch wird dann in 1,5 - 2 h bei max. +10°C in einen mit 400 kg Eis und 500 1 Wasser beladenen 1200 1-Email-Rührwerksapparat gegeben und noch ca. 30 min nachgerührt. Das ausgefallene Produkt wird abgeschleudert, mit Wasser gewaschen und getrocknet.
Ausbeute : 37 kg 4-Butyrylamino-3-methyl-5-nitrobenzoesäuremethylester (88 % d. Th.), das Produkt enthält ca. 8 - 10 % 4-Butyrylamino-3-methyl-6-nitrobenzoesäuremethylester und ca. 1 - 2 % 4-Butyrylamino-3-methyl-2-nitrobenzoesäuremethylester.

### Vergleichsbeispiel B

In einem 500 ml-Kolben werden 70,5 g Schwefelsäure 96 % vorgelegt und bei max. 20°C 23,5 g 4-Butyrylamino-3-methylbenzoesäuremethylester eingetragen. Danach werden bei -10 bis 0°C in ca. 60 min 36,2 g Mischsäure (ca. 65 % Schwefelsäure und 35 % Salpetersäure) zudosiert. Man rührt noch 60 min nach und gibt dann auf ein Gemisch aus 250 g Eis und 250 ml Wasser. Das ausgefallene Produkt wird abgesaugt, mit Wasser gewaschen und getrocknet. Ausbeute : 24,9 g 4-Butyrylamino-3-methyl-5-nitrobenzoesäuremethylester (89 % d. Th.), das Produkt enthält ca. 8 - 10 % 4-Butyrylamino-3-methyl-6-nitrobenzoesäuremethylester und ca. 1 - 2 % 4-Butyrylamino-3-methyl-2-nitrobenzoesäuremethylester.

## Patentansprüche

1. Hochregioselektives Nitrierverfahren zur Herstellung von Verbindungen der allgemeinen Formel I 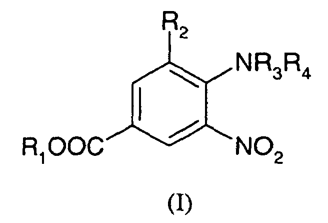 worin
R₁ H oder C₁ bis C₆-alkyl;
R₂ H oder C₁ bis C₆-alkyl;
R₃ H;
R₄ H oder C₁ bis C₅-alkyl-carbonyl bedeuten
oder R₃ und R₄ zusammen ein Succinyl-, Glutaryl- oder Adipinyl-Rest bedeuten können, **dadurch gekennzeichnet, daß** Verbindungen der Formel II 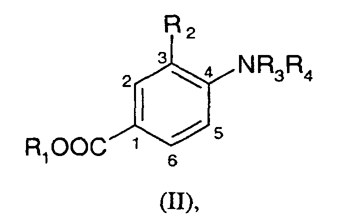 worin R₁, R₂, R₃ und R₄ die oben genannte Bedeutung aufweisen, in einem ersten Schritt bei weniger als 10°C in einem Lösungsmittelgemisch aus 1,5-2,5 mol Schwefelsäure, 1,5-2,5 mol Salpetersäure und 2 - 6 mol Wasser pro mol der Verbindung der allgemeinen Formel II gelöst wird und das Gemisch in einem zweiten Schritt bei - 20°C bis + 10°C mit 6 - 10 mol Salpetersäure pro mol der Verbindung der allgemeinen Formel II versetzt wird.

2. Nitrierverfahren gemäß Anspruch 1, **dadurch gekennzeichnet, daß** die Komponenten des Lösungsmittelgemisches des ersten Schritts in einem molaren Verhältnis von 1,8 - 2,2 mol Salpetersäure zu 1,8 - 2,2 mol Schwefelsäure zu 2,5 - 4 mol Wasser pro mol der Verbindung der allgemeinen Formel II stehen und im zweiten Schritt 7 - 9 mol Salpetersäure pro mol der Verbindung der allgemeinen Formel II hinzugegeben werden.

3. Nitrierverfahren gemäß Anspruch 1 oder 2, **dadurch gekennzeichnet, daß** die Komponenten des Lösungsmittelgemisches des ersten Schritts in einem molaren Verhältnis von 2 mol Salpetersäure zu 2 mol Schwefelsäure zu 3 mol Wasser pro mol der Verbindung der Formel II stehen und im zweiten Schritt 8 mol Salpetersäure pro mol der Verbindung der Formel II hinzugegeben werden.

4. Nitrierverfahren gemäß einem der Ansprüche 1, 2 und 3, **dadurch gekennzeichnet, daß** der die Verbindung der Formel II im ersten Schritt bevorzugt bei -20 bis + 10°C in das Lösungsmittelgemisch eingetragen wird.

5. Nitrierverfahren gemäß einem der Ansprüche 1, 2, 3 und 4, **dadurch gekennzeichnet, daß** die Zugabe der Salpetersäure im zweiten Schritt bei einer Temperatur zwischen -5 und + 5°C erfolgt.

6. Nitrierverfahren insbesondere gemäß einem der vorangegangen Anspräche 1, 2, 3, 4 und 5, **dadurch gekennzeichnet, daß** die Reste R₁, R₂, R₃ und R₄ die folgende Bedeutung haben:
R₁ H, Methyl, Ethyl, Propyl, Isopropyl, Butyl, Isobutyl, Pentyl, Hexyl;
R₂ H, Methyl, Ethyl, Propyl, Isopropyl, Butyl, Isobutyl, Pentyl, Hexyl;
R₃ H;
R₄ H, Acetyl, Propionyl, Butyryl, Valeryl, Capryl,
oder R₃ und R₄ bilden zusammen einen Succinyl-, Glutaryl- oder Adipinyl-Rest.

7. Nitrierverfahren insbesondere gemäß einem der vorangegangen Ansprüche 1, 2, 3, 4, 5 und 6, **dadurch gekennzeichnet, daß** die Reste R₁, R₂, R₃ und R₄ die folgende Bedeutung haben:
R₁ Methyl;
R₂ Methyl;
R₃ H;
R₄ Butyryl

## Claims

1. Highly regioselective nitration process for preparing compounds of general Formula I 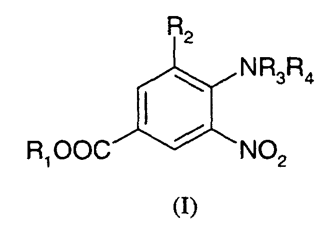 wherein
R₁ denotes H or C₁₋₆-alkyl;
R₂ denotes H or C₁₋₆-alkyl;
R₃ denotes H;
R₄ denotes H or C₁₋₅-alkyl-carbonyl,
or R₃ and R₄ together denote a succinyl-, glutaryl- or adipinyl- group, **characterised in that** a compound of Formula II 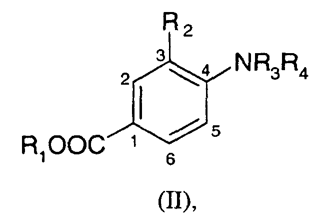 wherein R₁, R₂, R₃ and R₄ are as hereinbefore defined, is dissolved in a first step at less than 10°C in a solvent mixture of 1.5-2.5 mol of sulphuric acid, 1.5-2.5 mol of nitric acid and 2 - 6 mol of water per mol of the compound of general Formula II and in a second step at -20 to + 10°C the mixture is combined with 6 - 10 mol of nitric acid per mol of the compound of general Formula II.

2. Nitration process according to claim 1, **characterised in that** the components of the solvent mixture of the first step are in a molar ratio of 1.8 - 2.2 mol of nitric acid to 1.8 - 2.2 mol of sulphuric acid to 2.5 - 4 mol of water per mol of the compound of general Formula II and in the second step 7 - 9 mol of nitric acid are added per mol of the compound of general Formula II.

3. Nitration process according to claim 1 or 2, **characterised in that** the components of the solvent mixture of the first step are in a molar ratio of 2 mol of nitric acid to 2 mol of sulphuric acid to 3 mol of water per mol of the compound of Formula II and in the second step 8 mol of nitric acid are added per mol of the compound of Formula II.

4. Nitration process according to one of claims 1, 2 and 3, **characterised in that** the compound of Formula II is added to the solvent mixture in the first step preferably at -20 to + 10°C.

5. Nitration process according to one of claims 1, 2, 3, 4 and 5, **characterised in that** the nitric acid is added in the second step at a temperature of between -5 and +5°C.

6. Nitration process particularly according to one of the preceding claims 1, 2, 3, 4, and 5, **characterised in that** the groups R₁, R₂, R₃ and R₄ have the following meanings:
R₁ denotes H, methyl, ethyl, propyl, isopropyl, butyl, isobutyl, pentyl, hexyl;
R₂ denotes H, methyl, ethyl, propyl, isopropyl, butyl, isobutyl, pentyl, hexyl;
R₃ denotes H;
R₄ denotes H, acetyl, propionyl, butyryl, valeryl, capryl,
or R₃ and R₄ together form a succinyl, glutaryl or adipinyl group.

7. Nitration process particularly according to one of the preceding claims 1, 2, 3, 4, 5 and 6, **characterised in that** the groups R₁, R₂, R₃ and R₄ have the following meanings:
R₁ denotes methyl;
R₂ denotes methyl;
R₃ denotes H;
R₄ denotes butyryl.

## Revendications

1. Procédé de nitration hautement régiosélectif pour la préparation des composées de formule générale I 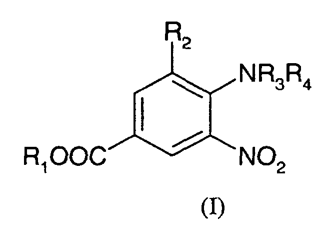 où
R₁ représente H ou C₁ à C₆-alkyle ;
R₂ représente H ou C₁ à C₆-alkyle ;
R₃ représente H ;
R₄ représente H ou C₁ à C₅-alkyl-carbonyle ;
ou R₃ et R₄ peuvent représenter ensemble un groupement succinyle, glutaryle ou adipinyle,
**caractérisé en ce que** une composée de formule II 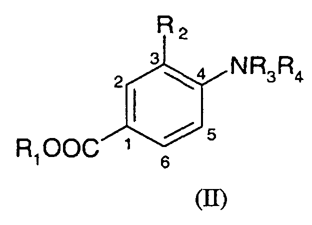 où R₁, R₂, R₃ et R₄ présentent la signification citée ci-dessus, est dissous dans une première étape à moins de 10°C dans un mélange de solvants de 1,5-2,5 moles d'acide sulfurique, 1,5-2,5 moles d'acide nitrique et 2-6 moles d'eau par mole de la composée de formule generale II et le mélange est additionné dans une seconde étape à -20°C à +10°C de 6-10 moles d'acide nitrique par mole de la composée de la formule generale II.

2. Procédé de nitration selon la revendication 1 **caractérisé en ce que** les composants du mélange de solvants de la première étape sont dans un rapport molaire de 1,8-2,2 moles d'acide nitrique à 1,8-2,2 moles d'acide sulfurique à 2,5-4 moles d'eau par mole de la composée de formule generale II et dans la seconde étape 7-9 moles d'acide nitrique par mole de la composée de formule generale II sont ajoutées.

3. Procédé de nitration selon la revendication 1 ou 2 **caractérisé en ce que** les composants du mélange de solvants de la première étape sont dans un rapport molaire de 2 moles d'acide nitrique à 2 moles d'acide sulfurique à 3 moles d'eau par mole de la composée de formule II et dans la seconde étape 8 moles d'acide nitrique par mole de la composée de formule II sont ajoutées.

4. Procédé de nitration selon l'une des revendications 1, 2 et 3
**caractérisé en ce que** la composée de formule II est introduit dans la première étape de préférence à -20 à +10°C dans le mélange de solvants.

5. Procédé de nitration selon l'une des revendications 1, 2, 3 et 4
**caractérisé en ce que** l'addition de l'acide nitrique dans la seconde étape a lieu à une température entre -5 et +5°C.

6. Procédé de nitration en particulier selon l'une des revendications 1, 2, 3, 4 et 5 précédentes **caractérisé en ce que** les groupements R₁, R₂, R₃ et R₄ ont la signification suivante :
R₁ représente H, méthyle, éthyle, propyle, isopropyle, butyle, isobutyle, pentyle, hexyle ;
R₂ représente H, méthyle, éthyle, propyle, isopropyle, butyle, isobutyle, pentyle, hexyle ;
R₃ représente H ;
R₄ représente H, acétyle, propionyle, butyryle, valéryle, capryle ;
ou R₃ et R₄ forment ensemble un groupement succinyle, glutaryle ou adipinyle.

7. Procédé de nitration en particulier selon l'une des revendications 1, 2, 3, 4, 5 et 6 précédentes **caractérisé en ce que** les groupements R₁, R₂, R₃ et R₄ ont la signification suivante :
R₁ représente méthyle ;
R₂ représente méthyle ;
R₃ représente H;
R₄ représente butyryle.
